Europäisches Patentamt

European Patent Office

Office européen des brevets

(19)

(11) **EP 0 814 058 B1**

(12) **EUROPÄISCHE PATENTSCHRIFT**

(45) Veröffentlichungstag und Bekanntmachung des Hinweises auf die Patenterteilung:
**23.12.1998 Patentblatt 1998/52**

(51) Int. Cl.$^6$: **C01B 37/00**, C01B 39/00, B01J 29/03, B01J 29/04, C07B 33/00, C07D 301/12

(21) Anmeldenummer: **97106682.4**

(22) Anmeldetag: **23.04.1997**

(54) **Verfahren zur Herstellung von kristallinen mikro- und mesoporösen Metallsilicaten, verfahrensgemäss erhältliche Produkte und deren Verwendung**

Method for preparation of crystalline micro- and mesoporous metal silicates, products obtained according to the method and their use

Procédé de préparation de silicates de métaux micro- et mésoporeux cristallins, produits obtenus suivant le procédé et leur utilisation

(84) Benannte Vertragsstaaten:
**BE DE ES FR GB IT PT SE**

(30) Priorität: **19.06.1996 DE 19624340**

(43) Veröffentlichungstag der Anmeldung:
**29.12.1997 Patentblatt 1997/52**

(73) Patentinhaber:
**Degussa Aktiengesellschaft
60311 Frankfurt (DE)**

(72) Erfinder:
• **Hasenzahl, Steffen, Dr.
63457 Hanau (DE)**
• **Mangold, Helmut, Dr.
63517 Rodenbach (DE)**
• **Roland, Eckehart, Dr.
63486 Bruchköbel (DE)**
• **Scholz, Mario Dr.
63584 Gründau (DE)**
• **Thiele, Georg, Dr.
63452 Hanau (DE)**

(56) Entgegenhaltungen:
**EP-A- 0 292 363          EP-A- 0 299 430
EP-A- 0 311 983          FR-A- 2 582 639**

**Beschreibung**

Die Erfindung betrifft ein Verfahren zur Herstellung von kristallinen mikro- und mesoporösen Metallsilicaten, die aus Siliciumdioxid und einem oder mehreren Metalloxiden aufgebaut sind, verfahrensgemäß erhältliche Produkte und deren Verwendung.

Es sind verschiedene kristalline mikro- und mesoporöse Metallsilicate bekannt. Unter Mikroporen werden nach JUPAC-Definition solche mit einem Durchmesser von kleiner 2 nm, unter Mesoporen solche mit 2 bis 50 nm verstanden.

Kristalline Metallsilicate mit regelmäßigen Mikro- oder Mesoporen stellen teilweise äußerst wirksame Katalysatoren in unterschiedlichsten Anwendungsbereichen dar. Insbesondere mikroporöse Produkte der Zusammensetzung $(SiO_2)_{1-x}(TiO_2)_x$, in denen Titanatome einen Teil der Siliciumatome im Kristallgitter ersetzen, haben technische Bedeutung als Oxidationskatalysatoren erlangt. Die Strukturen dieser Metallsilicate sind in Abhängigkeit von den Ausgansprodukten und Herstellbedingungen unterschiedlich: So weist beispielsweise der sogenannte Titansilicalit-1 die MFI-, Titansilicalit-2 die MEL- und Titan-Beta-Zeolith die BEA-Kristallstruktur auf. Kristallstrukturtypen bekannter Zeolithe und Silicalite sind beschrieben in W.M. Meier, D.H. Olson, Atlas of Zeolite Structure Types, Butterworth-Heinemann, 1993. Bekannte Strukturen mit regelmäßigen Mesoporen sind die MCM-41- und die MCM-48-Struktur, beschrieben in C. T. Kresge et al., Nature, 359, (1992) 710 - 712. Einen Überblick über Strukturen mit regelmäßigen Mesoporen gibt außerdem S. Behrens, Angew. Chemie, 1996, 108 (5), 561 - 564.

Es ist bekannt, daß die katalytische Wirksamkeit gattungsgemäßer Metallsilicate maßgeblich von deren Phasenreinheit und der Morphologie und damit von den Herstellbedingungen abhängt. Beispielsweise nimmt die katalytische Aktivität von Titansilicalit durch andere titanhaltige Phasen, etwa $TiO_2$, sowie mit zunehmender Größe der Kristalle ab - siehe B. Notari in (a) Catal. Today 18 (1993), 163 und (b) Stud. Surf. Sci. Catal. 67 (1991), 243.

Gattungsgemäße Metallsilicate, etwa Titansilicalit-1, lassen sich durch Hydrothermalsynthese herstellen: Dabei werden zunächst eine Quelle für Silicium und eine Quelle für Titan, üblicherweise Tetraalkylorthosilicat und Tetraalkylorthotitanat, in Gegenwart eines strukturbestimmenden quartären Ammoniumkations, meist als quartäres Ammoniumhydroxid eingesetzt , und Wasser zu einem Gel kondensiert und anschließend das Gel unter hydrothermalen Bedingungen, meist über 100 °C und autogenem Druck, kristallisiert; der gebildete Feststoff wird abgetrennt, gewaschen, getrocknet und bei über 300 °C kalziniert. Störanfällig ist die Art des Eintrags der Ti-Komponente, so daß eine Verunreinigung des Produktes durch $TiO_2$ als fremder Phase oft nicht ausgeschlossen werden kann - loc. cit B. Notari (b). Anstelle eines quartären Ammoniumhydroxids kann gemäß EP 543 247 als Templat auch eine Kombination aus einem quartären Ammoniumsalz und einer Base, etwa Ammoniak , eingesetzt werden, wobei jedoch relativ große Kristalle erhalten werden. Mit Cetyltrimethylammoniumhydroxid als Templat kann gemäß US-Patent 5,198,203 ein mesoporöses Titansilicat mit MCM-41-Struktur hergestellt werden.

Als weitere Quelle für Silicium ist pyrogene Kieselsäure bekannt: Gemäß R. Kumar et al. in Stud. Surf. Sci. Catal. 84 (1994) 109 ist Titansilicalit-1 durch Hydrothermalsynthese aus pyrogener Kieselsäure und Tetrabutylorthotitanat erhältlich. Durch die Verwendung eines Tetraalkylorthotitanats besteht die Gefahr, ein Produkt mit ungenügender Phasenreinheit zu erhalten.

Gemäß EP 0 311 983 wird zur Herstellung von Titansilicaliten ein copräzipitiertes poröses $TiO_2$-$SiO_2$-Material, das amorph oder kristallin sein kann, mit einer Templatverbindung imprägniert und dann der Hydrothermalsynthese unterworfen. Anregungen, Copräzipitate mit anderen Metallen oder pyrogene Mischoxide einzusetzen, lassen sich diesem Dokument nicht entnehmen.

Bei bekannten Syntheseverfahren werden die gattungsgemäßen mikroporösen Metallsilicate in Form von Kristalliten mit einer Größe von meistens weniger als einem Mikrometer gebildet, die sich nur mit größerem Aufwand von einer Flüssigkeit abtrennen lassen. Für viele technische Anwendungen wird daher das feine Material durch einen nachgeschalteten Agglomerationsschritt vergröbert. Gemäß EP 0 265 018 erfolgt die Agglomerierung unter Verwendung von oligomerem $SiO_2$ als Bindemittel.

Gemäß EP 0 299 430 wird eine vorgeformte amorphe $SiO_2$-Matrix mit einer wäßrigen Lösung, die eine lösliche Titanverbindung und ein geeignetes Templat enthält, imprägniert und unter hydrothermalen Bedingungen kristallisiert. Dabei bleiben Form und Größe der $SiO_2$-Matrix im wesentlichen erhalten. Auch dieses Verfahren hat den Nachteil, daß durch die Verwendung einer löslichen Titanverbindung die Gefahr besteht, daß die Phasenreinheit und damit die katalytische Aktivität gemindert werden.

Die Aufgabe der Erfindung besteht darin, ein weiteres Verfahren aufzuzeigen, das zu phasenreinen gattungsgemäßen Metallsilicaten mit hoher katalytischer Aktivität führt. Das Verfahren sollte sich nicht nur zur Herstellung von Produkten auf der Basis von $(SiO_2)_{1-x}(TiO_2)_x$ eignen, sondern auch zur Herstellung von Produkten, in welchen anstelle Titan ein oder mehrere andere Metalle im Kristallgitter eingebaut sind. Schließlich sollte das Verfahren gemäß einer besonderen Ausgestaltung auch so geführt werden können, daß unmittelbar, das heißt, ohne einen nachgeschalteten Agglomerationsschritt, bindemittelfreie Formkörper der kristallinen mikro- und mesoporösen Metallsilicate erhalten werden können.

Gefunden wurde ein Verfahren zur Herstellung von mikro- und mesoporösen Metallsilicaten, umfassend hydrothermale Umsetzung einer Quelle für Silicium in Gegenwart einer Quelle für das Metall und eines Templats, das dadurch gekennzeichnet ist ,daß man als Quelle für Silicium und das Metall ein pyrogenes Metall-Silicium-Mischoxid einsetzt.

Die allgemeine Zusammensetzung der pyrogenen Mischoxide und damit auch der herzustellenden kristallinen mikro- und mesoporösen Metallsilicate ist üblicherweise

$$(SiO_2)_{1-x}(A_mO_n)_x \qquad\qquad (Ia),$$

$$(SiO_2)_{1-x}((A_mO_n)_{1-y}(A'_{m'}O_{n'})_y)_x \qquad\qquad (Ib).$$

A und A' stehen für ein Metall der Wertigkeit p aus der Reihe B, Al, Ga, In, Ge, Sn, Pb oder der 3. bis 8. Nebengruppe.

m und m' sowie n und n'stehen für die Atomzahl, wobei $m \cdot p = 2n$ und $m' \cdot p = 2n'$ ist.

x steht für eine Zahl zwischen 0,0001 und 0,25, vorzugsweise zwischen 0,001 und 0,2 und insbesondere zwischen 0,005 und 0,1.

y in der Formel (Ib) steht für eine Zahl zwischen größer 0 und kleiner 1.

Bevorzugt zu verwendende pyrogene Mischoxide enthalten außer $SiO_2$ ein oder mehrere Oxide aus der Reihe $Al_2O_3$, $B_2O_3$, $TiO_2$, $ZrO_2$, $V_2O_5$, $Cr_2O_3$ oder $Fe_2O_3$. Gegebenenfalls liegen Oxide der Nebengruppenelemente außer in der höchsten auch in einer niedrigeren Oxidationsstufe vor. Zur Herstellung gattungsgemäßer Metallsilicate, welche als Oxidationskatalysator Verwendung finden sollen, werden solche Mischoxide verwendet, welche frei von Aluminiumoxid sind; durch das erfindungsgemäße Verfahren werden hierbei Metallsilicalite erhalten. Zur Herstellung gattungsgemäßer Metallsilicate der allgemeinen Zusammensetzung

$$(SiO_2)_{1-x}(M_rA_mO_{n''})_x \qquad\qquad (II)$$

werden Mischoxide der allgemeinen Zusammensetzung (Ia) eingesetzt. M in der Zusammensetzung (II) steht für ein Kation der Wertigkeit q aus der Reihe der Alkali- oder Erdalkalimetalle, $H^+$, $NH_4^+$ oder $N(alkyl)_4^+$; in (II) gilt $m \cdot p + r \cdot q = 2n''$. M wird im erfindungsgemäßen Verfahren entweder über das Templat oder bei der Hydrothermalsynthese anwesendes Alkali- oder Erdalkalihydroxid oder Ammoniak in das Metallsilicat eingeführt. Nach der Kalzinierung eines gattungsgemäßen Metallsilicats der Zusammensetzung (II) steht M für ein Proton oder ein Alkali- oder Erdalkalimetallion. Die genannten Kationen M oder auch andere Kationen lassen sich aber auch nachträglich durch übliche Ionenaustauscherreaktionen in die mikro- und mesoporösen Metallsilicate einbauen.

Die im erfindungsgemäßen Verfahren zu verwendenden pyrogenen Mischoxide lassen sich in ansich bekannter Weise aus einem Gemisch einer flüchtigen Silicium- und einer oder mehrerer flüchtiger Metallverbindungen (Verbindungen von A und/oder A'), beispielsweise durch Flammenhydrolyse oder Lichtbogenverfahren, herstellen. Im erfindungsgemäßen Verfahren werden vorzugsweise durch Flammenhydrolyse hergestellte pyrogene Mischoxide des Siliciums, wie sie beispielsweise aus Ullmann's Encyklopädie der technischen Chemie, 4. Auflage, Band 21, Seiten 464 bis 465 (1982) und DE-A 36 11 449 bekannt sind, verwendet.

Im erfindungsgemäßen Verfahren lassen sich sowohl die pulverförmigen pyrogenen Mischoxide als auch Formkörper derselben einsetzen.

Die Formkörper des Metall-Silicium-Mischoxides können durch bekannte Verfahren, wie zum Beispiel Sprühgranulation, Wirbelschichtgranulation, Extrusion, Pelletierung oder Tablettierung hergestellt werden. In einer bevorzugten Ausführungsform der Erfindung können die Formkörper mittels Sprühgranulation, wie sie beispielsweise in DE-A 36 11 449 beschrieben wird, hergestellt werden.

Bei dem erfindungsgemäßen Verfahren wird das pyrogene Metall/Silicium-Mischoxid in Pulverform oder in Form eines hieraus hergestellten bindemittelfreien Formkörpers mit einer wäßrigen Lösung eines geeigneten Templats kontaktiert; bei Verwendung gröberer Mischoxid-Formkörper, etwa Extrudaten, handelt es sich üblicherweise um eine Imprägnierung. An diesen Schritt schließt sich, eventuell nach einer Zwischentrocknung, die hydrothermale Umsetzung an, wobei das pyrogene Metall-Silicium-Mischoxid in das erfindungsgemäße kristalline mikroporöse oder mesoporöse Metallsilicat überführt wird. Nach der hydrothermalen Umsetzung wird in ansich bekannter Weise das Templat aus dem Metallsilicat entfernt - üblicherweise durch Extraktion oder vorzugsweise durch Kalzinieren.

Als Templatverbindung eignen sich Amine mit einer oder mehreren Aminogruppen, Aminoalkohole oder tetrasubstituierte Ammoniumverbindungen. Besonders geeignet sind Tetraalkylammoniumverbindungen, insbesondere Tetraalkylammoniumhydroxid. Die Art der Alkylgruppen in den genannten Tetraalkylammoniumverbindungen ist für die Struktur der Poren von Bedeutung: Zur Herstellung erfindungsgemäßer mikroporöser Metallsilicate werden bevorzugt solche Tetraalkylammoniumverbindungen eingesetzt, in welchen Alkyl für Ethyl, Propyl und Butyl, vorzugsweise n-Propyl, steht. So können beispielsweise für die Herstellung erfindungsgemäßer Metallsilicate mit MFI-Struktur Tetra-n-propyl-ammoniumverbindungen, Metallsilicate mit MEL-Struktur Tetra-n-butyl-ammoniumverbindungen und Metallsilicate mit BEA-Struktur Tetraethylammoniumverbindungen eingesetzt werden. Zur Herstellung erfindungsgemäßer mesoporöser Metallsilicate werden vorzugsweise solche Tetraalkylammoniumverbindungen eingesetzt, welche Tensidcharakter haben; demgemäß enthalten diese Verbindungen eine Alkylgruppe oder eine Alkenylgruppe mit mindestens 7 C-Atomen in der längsten Kette. Besonders bevorzugt werden Ammoniumverbindungen der allgemeinen Formel $RNR'_3{}^+X^-$, worin R 12 bis 18 C-Atome enthält und vorzugsweise unverzweigt und gesättigt ist, R' 1 oder 2 C-Atome aufweist und X für das Anion, üblicherweise Hydroxid oder Halogenid, steht. Besonders geeignet zur Herstellung mesoporöser Strukturen sind Cetyltrimethylammoniumchlorid bzw. -bromid und Lauryltrimethylammoniumchlorid bzw. -bromid. Bei Verwendung von Tetraalkylammoniumhalogeniden als Templat wird der zum Kontaktieren/Imprägnieren verwendeten Templatlösung eine Base zugefügt, wie Ammoniak, ein primäres, sekundäres oder tertiäres Amin oder ein Alkalimetall- oder Erdalkalimetallhydroxid.

Üblicherweise wird die Templatverbindung in einem molaren Verhältnis von Templat zu $SiO_2$ 0,05 bis 2,0 eingesetzt. Zur Herstellung mikroporöser Metallsilicate wird ein Molverhältnis von Templat zu $SiO_2$ zwischen 0,05 und 1,0, insbesondere zwischen 0,10 und 0,45, bevorzugt. Zur Herstellung erfindungsgemäßer mesoporöser Metallsilicate mit MCM-41- bzw. MCM-48-Struktur wird ein Molverhältnis Templat : $SiO_2$ zwischen 0,05 und 2,0, insbesondere zwischen 0,1 und 0,8, bevorzugt.

Das Molverhältnis $OH^-/SiO_2$ liegt im allgemeinen zwischen 0,05 und 2,0, vorzugsweise zwischen 0,1 und 1,0. Bei der hydrothermalen Umsetzung liegt das Molverhältnis von $H_2O/SiO_2$ zwischen 1 und 200, vorzugsweise zwischen 4 und 100. Der Reaktionsmischung kann man gegebenenfalls Impfkristalle desselben Strukturtyps zusetzen. Bei Verwendung von Formkörpern aus pyrogenen Mischoxiden wird das Molverhältnis $H_2O/SiO_2$ im allgemeinen niedriger liegen als bei Verwendung von pyrogenen Mischoxiden, wie sie unmittelbar bei der Flammenhydrolyse erhalten werden.

Beim erfindungsgemäßen Verfahren zur Herstellung mikroporöser Metallsilicate wird die hydrothermale Umsetzung üblicherweise unter autogenem Druck bei einer Temperatur zwischen 100 und 220 °C, vorzugsweise zwischen 150 und 190 °C, durchgeführt. Die Herstellung erfindungsgemäßer mesoporöser Metallsilicate ist auch bei niedrigerer Temperatur möglich, so daß der übliche Temperaturbereich zwischen 25 und 200 °C, vorzugsweise zwischen 50 und 175 °C und insbesondere zwischen 70 und 150 °C, liegt. Die hydrothermale Umsetzung wird solange durchgeführt, bis bei der gegebenen Reaktionstemperatur das eingesetzte pyrogene Mischoxid vollständig in das erfindungsgemäße kristalline mikro- oder mesoporöse Metallsilicat überführt worden ist. Die Vollständigkeit der Umsetzung läßt sich mittels bekannter Methoden zur Strukturuntersuchung ermitteln. Unter optimierten Bedingungen liegt die Reaktionszeit üblicherweise zwischen 1 und 50 Stunden.

Zur Abtrennung der Templatverbindung aus dem bei der hydrothermalen Umsetzung erhaltenen Feststoff wird dieser eventuell nach einem zuvor durchgeführten Waschprozeß meistens zwischen 300 und 1000 °C kalziniert, wobei der Kalzinierungsschritt gleichzeitig eine Formgebung umfassen kann. Bei den erfindungsgemäßen mesoporösen Metallsilicaten kann die Templatverbindung, zumindest teilweise, auch durch Extraktion mit einem organischen Lösungsmittel entfernt werden.

Gemäß einer besonderen Ausführungsform des erfindungsgemäßen Verfahrens wird das pyrogene Metall-Silicium-Mischxoid, wie es etwa bei der Flammenhydrolyse erhalten wird, in einer wäßrigen Templatlösung suspendiert. Dabei können dieselben Molverhältnisse, wie oben angegeben, eingehalten werden. Diese Suspension wird sprühgetrocknet und das templathaltige Sprühgranulat bei erhöhter Temperatur, beispielsweise bei 175 °C, gegebenenfalls in einer Wasserdampfatmosphäre im Autoklaven behandelt. Die genannte Sprühtrocknung wird zweckmäßigerweise durch Einstellung der Temperatur der Trocknungsluft im Bereich von 100 bis 500 °C so durchgeführt, daß das erhaltene Granulat einen Wassergehalt von 5 bis 50 Gew.-% aufweist. Nach der hydrothermalen Umsetzung werden die erhaltenen Formkörper zur Entfernung des Templats bei einer Temperatur von 300 bis 1000 °C, bevorzugt von 400 bis 800 °C, kalziniert.

Das erfindungsgemäße Verfahren läßt sich reproduzierbar und einfach steuerbar durchführen. Durch die Verwendung der pyrogenen Mischoxide werden Probleme durch die Anwesenheit artfremder Phasen anwesender Metalloxide vermieden. Die nach dem erfindungsgemäßen Verfahren erhältlichen kristallinen mikro- und mesoporösen Metallsilicate zeichnen sich durch eine hohe katalytische Aktivität aus. Die gattungsgemäßen Metallsilicate lassen sich zudem nicht nur in Form feinstpulvriger Stoffe sondern auch in Form unterschiedlicher Formkörper erhalten. Bei Verwendung von Formkörpern aus pyrogenen Mischoxiden oder Bildung solcher Formkörper unmittelbar vor der hydrothermalen Umsetzung ist ein nachträglicher Agglomerationsschritt nicht notwendig. Zudem entfällt ein Zusatz katalytisch inaktiver Bindemittel. Ein weiterer Vorteil erfindungsgemäß erhältlicher Formkörper aus kristallinen mikro- und mesoporösen Metallsilicaten besteht darin, daß sich diese leichter aus Flüssgkeiten abfiltrieren lassen als ungeformte Katalysatoren, wodurch ihre Verwendung als Suspensionskatalysatoren erleichtert wird. Da pyrogene Mischoxide leicht verformt werden können, etwa zu Kugeln, Extrudaten, Pellets oder Tabletten, und im erfindungsgemäßen Verfahren die Form im wesentlichen erhalten bleibt, gelangt man unmittelbar zu derartigen Formkörpern, die als Katalysator in Festbettreaktoren eingesetzt werden können.

Aus der EP 0 311 983 B ist zwar bekannt, daß copräzipitiertes $TiO_2$-$SiO_2$-Material zur Herstellung von Titansilicaliten einsetzbar ist, jedoch handelt es sich bei den zu verwendenden Copräzipitaten um sehr poröse Produkte. Demgemäß war überraschend, daß unter Verwendung porenfreier pyrogener Mischoxide, wie sie im erfindungsgemäßen Verfahren zum Einsatz gelangen, kristalline mikro- und mesoporöse Metallsilicate in einfacher Weise erhältlich sind.

Die erfindungsgemäß erhältlichen kristallinen mikro- und mesoporösen Metallsilicate finden als solche vorzugsweise als Formkörper Anwendung als Katalysatoren. Titansilicalite und andere aluminiumfreie Produkte eignen sich insbesondere als Katalysatoren für Oxidationsreaktionen mit Wasserstoffperoxid oder organischen Hydroperoxiden. Beispiele sind die Verwendung von Titansilicalit-1 als Katalysator zur Umsetzung von Olefinen z.B. Propen mit Wasserstoffperoxid zu Epoxiden (EP 100 119), zur Umsetzung von Aromaten mit Wasserstoffperoxid zu Hydroxyaromaten (DE 31 35 559), zur Umsetzung von aliphatischen Kohlenwasserstoffen mit Wasserstoffperoxid zu Alkoholen und Ketonen (EP 376 453) und die Umsetzung von Cyclohexanon mit Wasserstoffperoxid und Ammoniak zu Cyclohexanonoxim (EP 208 311). Titansilicalit-2 wird als Katalysator zur Hydroxylierung von Phenol (J.S. Reddy, S. Sivasanker, P. Ratnasamy, J. Mol. Catal. 71 (1992) 373) und zur Umsetzung von Cyclohexanon mit Wasserstoffperoxid und Ammoniak zu Cyclohexanonoxim eingesetzt (J.S. Reddy, S. Sivasanker, P. Ratnasamy, J. Mol. Catal. 71 (1992) 383). Der Titan-Beta-Zeolith kann als Katalysator zur Umsetzung von Olefinen mit Wasserstoffperoxid oder organischen Hydroperoxiden zu Epoxiden eingesetzt werden (A. Corma, P. Esteve, A. Martinez, S. Valencia, J. Catal. 152 (1995) 18 und EP 659 685).

**Beispiel 1: Herstellung eines Titansilicalit-1-Pulvers**

In einem Polyethylenbecherglas werden 137.0 g einer Tetrapropylammoniumhydroxidlösung (40 Gew.-%) und 229.8 g entionisertes Wasser vorgelegt und unter Rühren 111,1 g eines pyrogenen Silicium-Titan-Mischoxids mit 3,0 Gew.-% $TiO_2$ zugegeben. Das resultierende Synthesegel wird zunächst vier Stunden bei 80°C unter Rühren gealtert und anschließend 24 Stunden bei 175°C in einem Autoklaven kristallisiert. Der erhaltene Feststoff wird durch Zentrifugieren von der Mutterlauge abgetrennt, dreimal mit je 250 ml entionisiertem Wasser gewaschen, bei 90°C getrocknet und in Luftatmosphäre vier Stunden bei 550°C kalziniert. Die Ausbeute beträgt 98,3 g.

Das Röntgendiffraktogramm des so hergestellten Katalysators zeigt das für die MFI-Struktur typische Beugungsmuster, das IR-Spektrum die für titanhaltige Molekularsiebe charakteristische Bande bei 960 cm$^{-1}$. Die naßchemische Analyse ergibt einen Titangehalt von 2,3 Gew.-% $TiO_2$. Das DR-UV-Vis-Spektrum zeigt außerdem, daß die Probe frei von Titandioxid ist.

**Beispiel 2: Awendungsbeispiel zur Epoxdierung von Propylen mit Wasserstoffperoxid**

In einem thermostatisierten Laborautoklav mit Begasungsrührer werden 1.0 g des gemäß Beispiel 1 hergestellten Katalaysators in 300 ml Methanol bei 40 °C unter Propylenatmosphäre vorgelegt und das Lösungsmittel bei 3 bar Überdruck mit Propylen gesättigt. Dann werden unter Rühren 13.1 g 30 Gew.-% wäßrige Wasserstoffperoxidlösung in einer Portion zugegeben und die Reaktionsmischung bei 40 °C und 3 bar gehalten, wobei über einen Druckregler Propylen nachdosiert wird, um den Verbrauch durch die Reaktion auszugleichen. In regelmäßigen Abständen werden über ein Filter Proben entnommen und der Wasserstoffperoxidgehalt der Reaktionsmischung durch Redoxtitration mit Cer(IV)sulfat-Lösung bestimmt. Die Auftragung von $\ln(c/c_0)$ gegen die Zeit t, wobei c die gemessene $H_2O_2$-Konzentration zum Zeitpunkt t und $c_0$ die berechnete $H_2O_2$-Konzentration zu Beginn der Reaktion ist, ergibt eine Gerade. Aus der Steigung der Geraden wird mit der Beziehung

$$\frac{dc}{dt} = -k \cdot c \cdot c_{kat},$$

worin $c_{kat}$ für die Katalysatorkonzentration in kg Katalysator je kg Reaktionsmischung steht, die Aktivitätskennzahl k zu 25,4 min$^{-1}$ bestimmt.

**Beispiel 3: Herstellung eines Titansilicalit-1-Mikrogranulats**

In einem Polyethylen-Becherglas werden 800 g entionisiertes Wasser und 30 g Essigsäure vorgelegt und unter Rühren 200 g eines pyrogenen Silicium-Titanmischoxids mit 3.6 Gew.-% $TiO_2$ zugegeben. Diese Mischung wird fünf Minuten mit einem Ultraturax-Rührer geschert. Die resultierende Suspension wird über einen Sprühtrockner (NIRO-Atomizer Modell 1638; Eintrittstemperatur 380°C; Austrittstemperatur 102°C; Rotationsgeschwindigkeit der Zerstäuberscheibe 15000 min$^{-1}$) getrocknet. Von dem so erhaltenen Mikrogranulat mit einem mittleren Teilchendurchmesser von 30 μm werden 20 g mit einer Mischung aus 20 g einer Tetrapropylammoniumhydroxidlösung (40 Gew.-%) und 20 g entionisiertem Wasser imprägniert. Das so imprägnierte Mikrogranulat wird in einen Autoklaven mit Teflon-Inliner gefüllt und 24 Stunden bei 175°C unter statischen Bedingungen kristallisiert. Der erhaltene Feststoff wird durch Zentrifugieren von der Mutterlauge abgetrennt, dreimal mit je 100 ml entionisiertem Wasser gewaschen, bei 90°C getrocknet und in Luftatmosphäre vier Stunden bei 550 °C kalziniert. Die Ausbeute beträgt 17 g.

Bei dem so hergestellten Katalysator handelt es sich um ein Titansilicalit-1-Mikrogranulat.

**Beispiel 4: Herstellung eines Titansilicalit-1-Mikrogranulats**

In einem Polyethylenbecherglas werden 720,7 g entionisiertes Wasser und 179,3 g Tetrapropylammoniumhydroxidlösung (40 Gew.-%) vorgelegt und unter Rühren 100 g eines pyrogenen Siliciun-Titanmischoxids mit 3.6 Gew.-% $TiO_2$ zugegeben. Diese Mischung wird fünf Minuten mit einem Ultraturax-Rührer geschert. Die resultierende Suspension wird über einen Sprühtrockner (NIRO-Atomizer Modell 1638; Eintrittstemperatur 380°C; Austrittstemperatur 90°C; Rotationsgeschwindigkeit der Zerstäuberscheibe 15000 $min^{-1}$) getrocknet. Von dem so erhaltenen Mikrogranulat mit einem mittleren Teilchendurchmesser von 30 $\mu$m, einem Trockenverlust von 23 Gew-% (10 h bei 105°C; entspricht dem Wassergehalt) und einem Kalzinierverlust von 43 Gew.-% (5 h bei 550°C; entspricht dem Wasser- und Templatgehalt) werden 20 g in einem Autoklaven mit Teflon-Inliner gefüllt und 4 Tage bei 180°C unter statischen Bedingungen kristallisiert. Der Feststoff wird anschließend bei 90°C getrocknet und in Luftatmosphäre vier Stunden bei 550 °C kalziniert.

Bei dem so hergestellten Katalysator handelt es sich um ein Titansilicalit-1-Mikrogranulat.

**Beispiel 5: Herstellung eines Titan-Beta-Zeolith-Pulvers**

Zu einem Polyethylenbecherglas werden 435.3 g einer Tetraethylammoniumhydroxidlösung (35 Gew.-%) und 236.3 g entionisiertes Wasser vorgelegt und 109.6 g eines pyrogenen Silicium-Titan-Mischoxids mit 3.0 Gew.-% $TiO_2$ zugegeben. Die resultierende Mischung wird vier Stunden bei 80°C gealtert und nach Abkühlen auf Raumtemperatur werden 12 g Beta-Zeolith-Impfkristalle mit einem $SiO_2/Al_2O_3$-Verhältnis von 27 zugesetzt. Dieses Synthesegel wird 3 Tage bei 150°C und Rühren in einem Autosklaven kristallisiert. Der erhaltene Feststoff wird durch Zentrifugieren von der Mutterlauge abgetrennt, dreimal mit je 250 ml entionisiertem Wassser gewaschen, bei 90°C getrocknet und in Luftatmosphäre vier Stunden bei 550°C kalziniert. Das Röntgendiffraktogramm des so hergestellten Katalysators zeigt das für die BEA-Struktur typische Beugungsmuster. Die naßchemische Analyse ergibt einen Titangehalt von 1,8 Gew.-% $TiO_2$.

**Beispiel 6: Herstellung eines mesoporösen Titansilicats mit MCM-41-Struktur**

In einem Polyethylenbecherglas werden 114.2 g einer Tetramethylammoniumhydroxidlösung (10 Gew.-%) vorgelegt und unter Rühren 30.0 g eines pyrogenen Silicium-Titan-Mischoxids mit 3.6 Gew.-% $TiO_2$ zugegeben. Diese Mischung wird eine Stunde gerührt. Dann wird unter Rühren eine Suspension von 29.1 g Cetyltrimethylammoniumbromid ($C_{16}H_{33}(CH_3)_3NBr$) in 109.2 g entionisiertem Wasser zugegeben und weiter 10 Minuten gerührt. Das resultierende Synthesegel (molare Zusammensetzung: $SiO_2$ : 0.17 $C_{16}H_{33}(CH_3)_3N^+$ : 0.26 $Me_4NOH$ : 25 $H_2O$) wird 48 Stunden bei 140°C unter statischen Bedingungen in einem Autoklaven kristallisiert. Der erhaltenen Feststoff wird abfiltriert und mit entionisertem Wasser so lange gewaschen, bis das Filtrat neutral ist. Der Feststoff wird bei 90°C getrocknet und anschließend in Luftatmosphäre vier Stunden bei 640°C kalziniert. Die Ausbeute beträgt 28 g.

Das Röntgendiffraktogramm des so hergestellten Katalysators zeigt das für MCM-41-Materialien typische Beugungsmuster mit einem $d_{100}$ Reflex bei 3.60 nm. Aus der sorptionsanalytischen Untersuchung mit Stickstoff bei 77 K folgen ein mittlerer Porendurchmesser von 3.16 nm, eine BET-Oberfläche von 1040 $m^2 \ g^{-1}$ und ein Porenvolumen von 0.89 g $ml^{-1}$. Die naßchemische Analyse ergibt einen Titangehalt von 4.6 Gew.-% $TiO_2$. Das Material ist in der Epoxidierung von Propen mit Wasserstoffperoxid katalytisch aktiv.

**Beispiel 7: Herstellung eines mesoporösen Titansilicats mit MCM-48-Struktur**

In einem Polyethylenbecherglas werden 114.2 g einer Tetramethylammoniumhydroxidlösung (10 Gew.-%) vorgelegt und unter Rühren 30.0 g eines pyrogenen Silicium-Titan-Mischoxids mit 3.6 Gew.-% $TiO_2$ zugegeben. Diese Mischung wird eine Stunde gerührt. Dann wird unter Rühren eine Suspension von 114.2 g Cetyltrimethylammoniumbromid ($C_{16}H_{33}(CH_3)_3NBr$) in 435.2 g entionisiertem Wasser zugegeben und weiter 10 Minuten gerührt. Das resultierende Synthesegel (molare Zusammensetzung: $SiO_2$ : 0.65 $C_{16}H_{33}(CH_3)_3NBr$ : 0.26 $Me_4NOH$ : 62 $H_2O$) wird 48 Stunden bei 140°C unter statischen Bedingungen in einem Autoklaven kristallisiert. Der erhaltenen Feststoff wird abfiltriert und mit entionisertem Wasser so lange gewaschen, bis das Filtrat neutral ist. Der Feststoff wird bei 90°C getrocknet und anschließend in Luftatmosphäre vier Stunden bei 640°C kalziniert.

Bei dem so hergestellten Katalysator handelt es sich um Ti-MCM-48.

**Beispiel 8: Herstellung eines Bor-Pentasilzeoliths**

In einem Polyethylenbecherglas werden 800 g einer wäßrigen Hexamethylendiaminlösung (50 Gew.-%) vorgelegt und unter Rühren 70.7 g eines pyrogenen Silicium-Bor-Mischoxids mit 9.5 Gew.-% $B_2O_3$ zugegeben. Diese Mischung wird eine halbe Stunde gerührt und anschließend fünf Tage bei 170°C in einem Autoklaven kristallisiert. Der erhaltene Feststoff wird durch Zentrifugieren von der Mutterlauge abgetrennt, dreimal mit je 250 ml entionisiertem Wasser gewaschen, bei 90°C getrocknet und in Luftatmosphäre sechs Stunden bei 550°C kalziniert.

Das Röntgendiffraktogramm des so hergestellten Katalysators zeigt das für die MFI-Struktur typische Beugungsmuster. Die naßchemische Analyse ergibt einen $B_2O_3$-Gehalt von 2,4 Gew.-%.

**Beispiel 9: Herstellung eines Borsilicats mit MCM-41-Struktur**

In einem Polyethylen-Becherglas werden 157.6 g einer Tetramethylammoniumhydroxidlösung (10 Gew.-%) vorgelegt und unter Rühren 42.3 g eines pyrogenen Silicium-Bor-Mischoxids mit 5.5 Gew.-% $B_2O_3$ zugegeben. Diese Mischung wird eine Stunde gerührt. Dann wird unter Rühren eine Suspension von 65.5 g Cetyltrimethylammoniumbromid ($C_{16}H_{33}(CH_3)_3NBr$) in 576.5 g entionisiertem Wasser zugegeben und weiter 10 Minuten gerührt. Durch Zugabe von verdünnter Schwefelsäure wird der pH-Wert auf 11.5 eingestellt. Das resultierende Synthesegel wird 48 Stunden bei 140°C unter statischen Bedingungen in einem Autoklaven kristallisiert. Der erhaltenen Feststoff wird abfiltriert und mit entionisertem Wasser so lange gewaschen, bis das Filtrat neutral ist. Der Feststoff wird bei 90°C getrocknet und anschließend in Luftatmosphäre vier Stunden bei 540°C kalziniert.

Das Röntgendiffraktogramm des erhaltenen Katalysators zeigt das für die MCM-41 Materialien typische Beugungsmuster. Aus der sorptionsanalytischen Untersuchung mit Stickstoff bei 77°K folgen ein mittlerer Porendurchmesser von 2,83 nm, eine BET-Oberfläche von 1060 $m^2$/g und ein Porenvolumen von 0,89 ml/g.

**Beispiel 10: Herstellung eines Zirkonsilicalit-1-Pulvers**

In einem Polyethylenbecherglas werden 62.4 g einer Tetrapropylammoniumhydroxidlösung (40 Gew.-%) und 229.8 g entionisertes Wasser vorgelegt und unter Rühren 57.4 g eines pyrogenen Silicium-Zirkon-Mischoxids mit 3.0 Gew.-% $ZrO_2$ zugegeben. Das resultierende Synthesegel wird anschließend 24 Stunden bei 175°C in einem Autoklaven kristallisiert. Der erhaltene Feststoff wird durch Zentrifugieren von der Mutterlauge abgetrennt, dreimal mit je 250 ml entionisiertem Wasser gewaschen, bei 90°C getrocknet und in Luftatmosphäre vier Stunden bei 550°C kalziniert.

Das Röntgendiffraktogramm des erhaltenen Katalysators zeigt das für die MFI-Struktur typische Beugungsmuster. Die Röntgenfluoreszenzanalyse ergibt einen $ZrO_2$-Gehalt von 1,0 Gew.-%.

**Beispiel 11: Herstellung eines Zirkonsilicats mit MCM-41-Struktur**

In einem Polyethylen-Becherglas werden 114.2 g einer Tetramethylamnoniumhydroxidlösung (10 Gew.-%) vorgelegt und unter Rühren 30.0 g eines pyrogenen Silicium-Zirkon-Mischoxids mit 3.0 Gew.-% $ZrO_2$ zugegeben. Diese Mischung wird eine Stunde gerührt. Dann wird unter Rühren eine Suspension von 29.1 g Cetyltrimethylammoniumbromid ($C_{16}H_{33}(CH_3)_3NBr$) in 109.2 g entionisiertem Wasser zugegeben und weiter 10 Minuten gerührt. Das resultierende Synthesegel wird 48 Stunden bei 140°C unter statischen Bedingungen in einem Autoklaven kristallisiert. Der erhaltenen Feststoff wird abfiltriert und mit entionisertem Wasser so lange gewaschen, bis das Filtrat neutral ist. Der Feststoff wird bei 90°C getrocknet und anschließend in Luftatmosphäre vier Stunden bei 640°C kalziniert.

Das Röntgendiffraktogramm des erhaltenen Katalysators zeigt das für MCM-41-Materialien typische Beugungsmuster mit einem $d_{100}$-Wert von 3.25 nm. Aus der sorptionsanalytischen Untersuchung mit Stickstoff bei 77°K folgen ein mittlerer Porendurchmesser von 2,4 nm, eine BET-Oberfläche von 860 $m^2$/g und ein Porenvolumen von 0,59 ml/g.

**Patentansprüche**

1. Verfahren zur Herstellung von mikro- und mesoporösen Metallsilicaten, umfassend hydrothermale Umsetzung einer Quelle für Silicium und das Metall in Gegenwart eines Templats,
dadurch gekennzeichnet,
daß man als Quelle für Silicium und das Metall ein pyrogenes Metall-Silicium-Mischoxid einsetzt.

2. Verfahren nach Anspruch 1 zur Herstellung von kristallinen mikro- und mesoporösen Metallsilicaten der allgemeinen Zusammensetzung

$$(SiO_2)_{1-x}(A_mO_n)_x \qquad\qquad \text{(Ia)},$$

$$(SiO_2)_{1-x}((A_mO_n)_{1-y}(A'_{m'}O_{n'})_y)_x \qquad\qquad \text{(Ib)},$$

oder

$$(SiO_2)_{1-x}(M_rA_mO_{n''})_x \qquad\qquad (II),$$

worin bedeuten:

| | |
|---|---|
| x: | eine Zahl zwischen 0,0001 und 0,25; |
| y: | eine Zahl zwischen größer 0 und kleiner 1; |
| A und A': | ein Metall der Wertigkeit p aus der Reihe B, Al, Ga, In, Ge, Sn, Pb oder der 3. bis 8. Nebengruppe; |
| M: | ein Kation der Wertigkeit q aus der Reihe der Alkali- oder Erdalkalimetalle, $H^+$, $NH_4^+$ oder $N(alkyl)_4^+$; |
| m, m', n, n', n'' und r: | Atomzahl, wobei gilt: $m \cdot p = 2n$ und $m' \cdot p = 2n'$ sowie $m \cdot p + r \cdot q = 2n''$, |

dadurch gekennzeichnet,
daß man ein pyrogenes Mischoxid der Zusammensetzung (Ia) oder (Ib) einsetzt und das Kation M der Formel (II) über das Templat oder bei der Hydrothermalsynthese anwesendes Alkali- oder Erdalkalihydroxid einbringt.

3. Verfahren nach Anspruch 1 oder 2,
dadurch gekennzeichnet,
daß man ein durch Flammenhydrolyse hergestelltes pyrogenes Metall-Silicium-Mischoxid einsetzt.

4. Verfahren nach einem der Ansprüche 1 bis 3,
dadurch gekennzeichnet,
daß man ein pyrogenes Mischoxid aus der Reihe $(SiO_2)_{1-x}(TiO_2)_x$, $(SiO_2)_{1-x}(Al_2O_3)_x$, $(SiO_2)_{1-x}(B_2O_3)_x$, $(SiO_2)_{1-x}(V_2O_5)_x$, $(SiO_2)_{1-x}(ZrO_2)_x$ einsetzt

5. Verfahren nach einem der Ansprüche 1 bis 4,
dadurch gekennzeichnet,
daß man das Templat in einer Menge von 0,05 bis 2,0 Mol pro Mol $SiO_2$ im Mischoxid einsetzt.

6. Verfahren nach einem der Ansprüche 1 bis 5,
dadurch gekennzeichnet,
daß man die hydrothermale Umsetzung bei der Herstellung von mikroporösen Metallsilicaten bei einer Temperatur zwischen 100 bis 220 °C, vorzugsweise 150 bis 190 °C, und bei der Herstellung von mesoporösen Metallsilicaten zwischen 50 bis 175 °C, vorzugsweise 70 bis 150 °C, unter mindestens autogenem Druck durchführt.

7. Verfahren nach einem der Ansprüche 1 bis 6,
dadurch gekennzeichnet,
daß man das pyrogene Mischoxid mit einer wäßrigen, Hydroxylionen enthaltenden Templatlösung kontaktiert, wobei das Molverhältnis von $OH^-$ zu $SiO_2$ zwischen 0,05 bis 2,0 liegt.

8. Verfahren nach einem der Ansprüche 1 bis 7,
dadurch gekennzeichnet,
daß man das pyrogene Silicium-Metall-Mischoxid in Form eines Formkörpers einsetzt, wobei Größe und Form der Formkörper bei der hydrothermalen Umsetzung zu mikro- und mesoporösen Metallsilicaten im wesentlichen erhalten bleiben.

9. Bindemittelfreie Formkörper aus mikro- und mesoporösen Metallsilicaten, erhältlich nach dem Verfahren des Anspruches 8.

10. Verwendung der Formkörper gemäß Anspruch 9, worin die Zusammensetzung einem Al-freien Metallsilicalit entspricht, als Katalysator bei Oxidationsreaktionen.

11. Verwendung der Metallsilikate, hergestellt nach dem Verfahren gemäß den Ansprüchen 1 bis 8 als Katalysator bei der Oxidation von Olefinen, wie z.B. Propen, mit Wasserstoffperoxid, zu den entsprechenden Epoxiden.

**Claims**

1. Process for the production of micro- and mesoporous metallosilicates, comprising hydrothermal reaction of a source of silicon and the metal in the presence of a template,
characterised in that
a pyrogenic metal/silicon mixed oxide is used as the source of silicon and the metal.

2. Process according to claim 1 for the production of crystalline micro- and mesoporous metallosilicates of the general composition

$$(SiO_2)_{1-x}(A_mO_n)_x \qquad\qquad (Ia),$$

$$(SiO_2)_{1-x}((A_mO_n)_{1-y}(A'_{m'}O_{n'})_y)_x \qquad\qquad (Ib),$$

or

$$(SiO_2)_{1-x}(M_rA_mO_{n''})_x \qquad\qquad (II),$$

in which

x means
a number between 0.0001 and 0.25;
y means
a number between greater than 0 and less than 1;
A and A' mean
a metal of valency p from the range comprising B, Al, Ga, In, Ge, Sn, Pb or from sub-groups 3 to 8;
M means
a cation of valency q from the range comprising alkali or alkaline earth metals, $H^+$, $NH_4^+$ or $N(alkyl)_4^+$;
m, m', n, n', n'' and r mean
the atomic number, wherein the following applies:
$m \cdot p = 2n$ and $m' \cdot n = 2n'$ and
$m \cdot p + r \cdot q = 2n''$,

characterised in that
a pyrogenic mixed oxide of the composition (Ia) or (Ib) is used and the cation M of the formula (II) is introduced by means of the template or the alkali or alkaline earth metal hydroxide present during hydrothermal synthesis.

3. Process according to claim 1 or 2,
characterised in that
a pyrogenic metal/silicon mixed oxide produced by flame hydrolysis is used.

4. Process according to one of claims 1 to 3,
characterised in that
a pyrogenic mixed oxide from the range comprising $(SiO_2)_{1-x}(TiO_2)_x$, $(SiO_2)_{1-x}(Al_2O_3)_x$, $(SiO_2)_{1-x}(B_2O_3)_x$, $(SiO_2)_{1-x}(V_2O_5)_x$, $(SiO_2)_{1-x}(ZrO_2)_x$ is used.

5. Process according to one of claims 1 to 4,
characterised in that
the template is used in a quantity of 0.05 to 2.0 mol per mol of $SiO_2$ in the mixed oxide.

6. Process according to one of claims 1 to 5,
characterised in that
the hydrothermal reaction is performed at a temperature of between 100 to 220°C, preferably of 150 to 190°C, during production of microporous metallosilicates, and between 50 to 175°C, preferably of 70 to 150°C, during production of mesoporous metallosilicates, under at least autogenous pressure.

7. Process according to one of claims 1 to 6,
characterised in that

the pyrogenic mixed oxide is brought into contact with an aqueous template solution containing hydroxyl ions, wherein the molar ratio of OH⁻ to $SiO_2$ is between 0.05 to 2.0.

8. Process according to one of claims 1 to 7,
characterised in that
the pyrogenic silicon/metal mixed oxide is used in the form of a shaped article, wherein the size and shape of the shaped article during the hydrothermal reaction to yield micro- and mesoporous metallosilicates are substantially retained.

9. Binder-free shaped articles of micro- and mesoporous metallosilicates obtainable using the process of claim 8.

10. Use of the shaped articles according to claim 9, in which the composition corresponds to an Al-free metallosilicalite, as a catalyst in oxidation reactions.

11. Use of the metallosilicates produced using the process according to claims 1 to 8 as a catalyst in the oxidation of olefins, such as for example propene, with hydrogen peroxide to yield the corresponding epoxides.

## Revendications

1. Procédé de préparation de silicates de métaux micro- et mésoporeux, comprenant la conversion hydrothermique d'une source de silicium et de métal en présence d'un gabarit,
caractérisé en ce
qu'on utilise comme source de silicium et de métal un oxyde pyrogène mixte de métal et de silicium.

2. Procédé selon la revendication 1 pour la préparation de silicates cristallins de métaux micro- et mésoporeux de composition générale :

$$(SiO_2)_{1-x}(A_mO_n)_x \ (Ia), \ (SiO_2)_{1-x}((A_mO_n)_{1-y}(A'_{m'}O_{n'})_y)_x \ (Ib)$$

ou

$$(SiO_2)_{1-x}(M_rA_mO_{n''})_x \ (II),$$

dans laquelle :

x est un nombre compris entre 0,0001 et 0,25;
y est un nombre plus grand que zéro et plus petit que 1;
A et A' représentent un métal de valence p de la série B, Al, Ga, In, Ge, Sn, Pb ou du 3ième au 8ième sous-groupes;
M est un cation de valence q de la série des métaux alcalins ou alcalino-terreux, $H^+$, $NH_4^+$ ou $N(alkyle)_4^+$; m, m', n, n', n'' et r représentent un nombre d'atomes tel que m.p = 2n et m'.p = 2n', et m.p + r.q = 2n'',

caractérisé en ce
qu'on utilise un oxyde pyrogène mixte de composition (Ia) ou (Ib) et on introduit le cation M de formule (II) via le gabarit ou l'hydroxyde de métal alcalin ou alcalino-terreux présent dans la synthèse hydrothermique.

3. Procédé selon la revendication 1 ou 2,
caractérisé en ce
qu'on utilise un oxyde pyrogène mixte de métal et de silicium préparé par hydrolyse à la flamme.

4. Procédé selon l'une quelconque des revendications 1 à 3,
caractérisé en ce
qu'on utilise un oxyde pyrogène mixte de la série $(SiO_2)_{1-x}(TiO_2)_x$, $(SiO_2)_{1-x}(Al_2O_3)_x$, $(SiO_2)_{1-x}(B_2O_3)_x$, $(SiO_2)_{1-x}(V_2O_5)_x$ et $(SiO_2)_{1-x}(ZrO_2)_x$.

5. Procédé selon l'une quelconque des revendications 1 à 4,
caractérisé en ce
qu'on utilise le gabarit en quantité de 0,05 à 2,0 moles par mole de $SiO_2$ dans l'oxyde mixte.

6. Procédé selon l'une quelconque des revendications 1 à 5,
caractérisé en ce
qu'on réalise la conversion hydrothermique lors de la préparation de silicates de métaux microporeux à une température entre 100 et 220°C, de préférence de 150 à 190°C, et lors de la préparation de silicates de métaux mésoporeux, entre 50 et 175°C, de préférence de 70 à 150°C, sous pression au moins autogène.

7. Procédé selon l'une quelconque des revendications 1 à 6,
caractérisé en ce
qu'on met en contact l'oxyde pyrogène mixte avec une solution de gabarit aqueuse contenant des ions hydroxyle, le rapport molaire de $OH^-$ au $SiO_2$ se situant entre 0,05 et 2,0.

8. Procédé selon l'une quelconque des revendications 1 à 7,
caractérisé en ce
qu'on utilise l'oxyde pyrogène mixte de silicium et de métal sous la forme d'un corps moulé dont la dimension et la forme restent sensiblement inchangées lors de la conversion hydrothermique en silicates de métaux micro- et mésoporeux.

9. Corps moulé sans liant constitué de silicates de métaux micro- et mésoporeux, qui peut être obtenu par le procédé de la revendication 8.

10. Utilisation des corps moulés selon la revendication 9, dont la composition correspond à un silicalite de métal sans Al, comme catalyseur dans des réactions d'oxydation.

11. Utilisation des silicates de métaux préparés par le procédé selon les revendications 1 à 8 comme catalyseur dans l'oxydation d'oléfines, par exemple de propène, avec du peroxyde d'hydrogène, en époxydes correspondants.